# EUROPEAN PATENT APPLICATION

(11) **EP 1 629 852 A2**
(43) Date of publication of application: **01.03.2006**
(21) Application number: 05017480.4
(22) Date of filing: 11.08.2005
(51) Int. Cl.: A61K 47/02, A61K 47/18, A61K 47/38

(54) **Topical anaesthesia formulation for bodily cavities**

(30) Priority: 17.08.2004 US 602501 P; 06.12.2004 US 5618
(71) Applicant: Hystagel, Inc., Los Altos CA 94022 (US)
(72) Inventor: Vancaillie, Thierry G., Castlecrag New South Wales 2068 (AU); Hewitt, Alan Ernest, Windsor Downs New South Wales 2754 (AU)
(74) Representative: Käck, Jürgen

(57) **Abstract**

For use in bodily cavities, a topical anesthetic combination having a pH adjusted to optimize the effectiveness of the anesthetic, a viscosity appropriate to displace tissue debris and enhance retention, and addition of chemicals which increase the bio-availability of the anesthetic molecules.

## Description

### Background

### Technical Field

This invention relates generally to topical anesthetics and more specifically to a combination of topical anesthetic ingredients having pH, viscosity, and bio-availability properties suitable for use in bodily cavities.

### Discussion of Prior Art

In their "natural" state, topical anesthetics such as Lidocaine, also known as Lignocaine, have a low (acidic) pH of 3.5 to 4.5, good solubility in aqueous solution, and potentially indefinite stability. Objectionably though, low pH anesthetics when applied to patients can be irritating and are not as active as when the pH is within the range of the human tissues.

In contrast, it has been observed that an anesthetic solution buffered to a pH of around neutral pH = 7 is less irritating and hence more patient acceptable. The onset of action of buffered anaesthetics may be more rapid (DiFazio et al. Anesthetic Analg. 1986 July: 65(7): 760-4) and more effective on prior inflamed tissue (Murakami et al, Journal Dermatology Surgical Oncology 1994).

However, raising the pH from 3.5 to neutral makes the anesthetic gradually less soluble and more likely to precipitate (Larson et al. Journal Dermatology Surgical Oncology 1991; 17:411-14 and Trisiel LA, "Stability of Compounded Formulations" 2nd ed 2000). When precipitated, the anesthetic molecules are not bio-available and have no anesthetic effect.

To compensate for the decrease in solubility caused by increasing pH, commercially available preparations of higher (neutral) pH -caine anesthetics limit their concentrations to a low (< 1%) percentage. This trade-off of concentration against pH maintains the anesthetic in solution. However, the active ingredients are subject to degradation in higher pH solutions. For example, lignocaine maintained at a pH of 7 even at low concentration can degrade 35% within 4 weeks. A low concentration of an anesthetic with tissue-adjusted pH may be satisfactory for injection right into the site of action, but not when the anesthetic molecules must penetrate through the mucosa to reach the site of action. For topical applications, anesthetics need high concentrations to promote penetration of the molecules toward the tissues. Low concentration preparations, even at a pH close to the target tissue, are not effective. Resorting to administering drugs intravenously causes major side effects.

There is therefore a need for a topically applied product which delivers anesthetic molecules at body-adjusted pH, and which maintains its bio-availability.

### Summary

The invention is defined in claims 1, 2, 12 and 20, respectively. Particular embodiments of the invention are set out in the dependent claims. The invention in a preferred embodiment includes pharmacological agents which can be used as topical anesthetic with 1) body-adjusted pH, 2) viscosity agents which provide the ability to displace blood and exudates without preventing the product from reaching all areas of the cavity and to increase retention of the product within the cavity, and 3) chemical agents which prevent crystallization of the active molecules to increase their availability for migration from the product toward the target tissue, which provides for optimal usage within bodily cavities.

### Detailed Description

An un-buffered solution of amide anesthetic can remain stable for years, but once it is buffered to make it more bio-active, the solution will remain stable for only a few days before it loses solubility, precipitates, and becomes useless. To mitigate the problem of insolubility following buffering high concentration anesthetics, the invention provides two components, an aqueous solution of anesthetic at low pH, and a buffering solution comprising preferably a mixed phosphate base. The invention stores the anesthetic component and the buffer component separately, which allows maximizing the buffer pH independently of the anesthetic concentration.

Mixing a high (>8) pH buffer solution with a high (>2%) concentration anesthetic solution produces a high (>1%) concentration aqueous solution of topical anesthetic having a pH around 7. This combination verges on instability: A small increase in pH would cause the active ingredient to become insoluble and to precipitate immediately, thereby dramatically reducing the bio-availability of the active ingredient.

To obtain a margin of solubility, preferably a mixed phosphate base (Potassium Dihydrogen Orthophosphate and Dipotassium Hydrogen Orthophosphate) buffering solution having a pH of 8 is used for the buffering component. Sodium Hydroxide may also be a suitable buffering component. A >2% concentration of pH 3.8 Lidocaine is preferably used for the anesthetic component, although other amide anesthetics may also be suitable. The pH and viscosity levels are set to reflect the target organ.

Ranges of pH and concentration in components and mixture:

| | Anesthetic Component | Buffering Component | Mixture |
|---|---|---|---|
| pH | 2.5 - 5 | 7-11 | 5.5 - 7 |
| Concentration of Anesthetic | > 2 % | 0 | > 1 % |

To delay coalescence and postpone precipitation of the ingredient molecules within the mixture of the two solutions, the two solutions are rapidly mixed together in a special technique called "speed mixing."

### Enhancer

Anesthetic compound molecules in a solution naturally tend to combine, crystallize, and precipitate as larger solids which can no longer penetrate through tissue to a target. A solubilizing agent (or "enhancer") in a solution inhibits crystallization, and thereby increases the solubility, of chemicals (e.g., anesthetic agents) in the solution. This enables the solution to hold a higher concentration of the anesthetic agent and thereby increases the bio-availability, potency, and effect of the anesthetic agent. Since anesthetic concentration is not lost, it does not need to begin at a higher than optimum level.

The invention in an embodiment having Lignocaine as the active ingredient and having a phosphate based buffer preferably incorporates into the buffer N-Methyl-2-Pyrrolidone as an enhancer. Afterwards, upon mixing the buffer and the anesthetic, any small quantities of drug that begin to crystallize are immediately solubilised and forestalled from acting as seed sites for precipitation.

### Viscosity

Topical anesthetics need to be applied directly to their site of action. Doing this may be relatively simple when the target tissue is visible to the physician and accessible without the need to pass body openings. However when the target tissue is within a body cavity, a major barrier is presented by the cumulative mass of secretions, tissue debris, blood, exudates, etc. In order to displace this mass, the viscosity of the anesthetic vehicle must be higher than that of blood and exudates, and high enough to help the solution adhere to uterine walls. A benefit of having a viscosity slightly higher than that of blood is that capillary force attracts the vehicle containing the anesthetic molecules to remain within the bodily cavity for the time needed for the anesthetic molecules to transfer from the liquid vehicle into the target tissue.

On the other hand the viscosity must not be much higher than that of blood, to preserve the solution's ability to flow easily and enter intended-to-be-anesthetized nooks and crannies of the bodily cavity that are inaccessible to more viscous gels. Viscous aqueous X-Ray solutions are known to spread to nooks and crannies of uterine cavities and into tubes, and to remain there for hours.

To obtain a suitable viscosity, the invention preferably adds Hydroxypropyl Methylcellulose in 50:50 proportions to both components. Various concentrations were tested to find a level that provided a good balance of properties stable at the natural and buffered pH. A concentration of 0.1% was found to provide a suitable viscosity. This agent effectively maintains the viscosity of the aqueous solution, and is generally unaffected by pH in the range of 3 to 11. Sonography has shown the invention, with a similar level of viscosity in an embodiment identified as Hystagel™, penetrating and being retained in uterine cavities for several hours after instillation.

Mixing is postponed until just prior to the procedure, and then done up to a number of hours prior to use. Mixing these anesthetic and buffering components in a 1:1 ratio produces a >1% concentration aqueous solution having a pH of around 7. This delivers the product with good bio-availability and effectiveness. Once mixed, the anesthetic is stable for several hours, and in terms of efficacy, ease of use, application and persistence it is very effective as a local anesthetic.

While the present invention is described in terms of a preferred embodiment, it will be appreciated by those skilled in the art that this embodiment may be modified without departing from the essence of the invention. It is therefore intended that the following claims be interpreted as covering any modifications falling within the true spirit and scope of the invention.

## Claims

1. A method of delivering a near neutral pH topical anesthetic agent in a mixture into a natural body cavity to cause a rapid onset anesthetic effect without precipitation of the anesthetic.

2. A method of making an anesthetic solution, comprising the steps of:
providing a buffering agent having a pH of between 7 and 11 in a buffering solution which, when mixed with a -caine anesthetic agent having a pH of between 2.5 and 5 , will yield a buffered anesthetic solution having a pH of at least 5.5;
providing an amide anesthetic agent having a pH in a range between about 3.5 and 4.5 at at least 10% concentration in an unbuffered solution; and
mixing the buffering solution and the unbuffered anesthetic solution to produce a buffered anesthetic solution having a pH of at least 5.5.

3. The method of claim 2 further comprising the step of adding a solubising agent to the buffering agent before the mixing step.

4. The method of claim 3 wherein the solubising agent comprises N-Methyl-2-Pyrrolidone.

5. The method of claim 2 further comprising the step of adding a viscosity agent to at least one of the buffering agent and the unbuffered solution.

6. The method of claim 5 wherein the viscosity agent comprises Hydroxypropyl Methylcellulose.

7. The method of claim 5 wherein the viscosity agent is added to both the buffering agent and the unbuffered solution.

8. The method of claim 7 wherein the viscosity agent is added in equal volumes to both the buffering agent and the unbuffered solution.

9. The method of claim 2 wherein the buffering solution comprises Potassium Dihydrogen Orhtophosphate.

10. The method of claim 2 wherein the buffering solution comprises Dipotassium Hydrogen Orthophosphate.

11. The method of claim 2 wherein the buffering solution comprises:
Potassium Dihydrogen Orhtophosphate, and
Dipotassium Hydrogen Orthophosphate.

12. A composition of matter comprising:
at least 2% by volume concentration of an amide anesthetic at a pH of 5 or less and having a viscosity greater than 1.

13. The composition of claim 12 wherein the amide anesthetic comprises Lidocaine.

14. The composition of claim 12 wherein the amide anesthetic comprises Marcaine.

15. The composition of claim 12 wherein the amide anesthetic comprises Carbocaine.

16. The composition of claim 12 further comprising a solubising agent.

17. The composition of claim 16 wherein the solubising agent comprises N-Methyl-2-Pyrrolidone.

18. The composition of claim 12 further comprising a viscosity agent.

19. The composition of claim 18 wherein the viscosity agent comprises hydroxypropyl methylcellulose.

20. A binary drug comprising:
a first component including
a buffering agent having a pH such that, when mixed with an amide anesthetic compound having a pH of at least 3.5, will yield a buffered anesthetic solution having a pH of at least 6;
a solubising agent; and
a viscosity agent; and
a second component including
an amide anesthetic agent having a pH in a range between about 3.5 and 4.5 at at least >2% concentration in aqueous solution; and
a viscosity agent.

21. The binary drug of claim 20 wherein the buffering agent comprises:
potassium dihydrogen orthophosphate or dipotassium hydrogen orthophosphate.

22. The binary drug of claim 20 wherein the solubising agent comprises N-Methyl-2-Pyrrolidone.

23. The binary drug of claim 20 wherein the viscosity agent comprises Hydroxypropyl Methylcellulose.
